(19) Europäisches Patentamt
European Patent Office
Office européen des brevets

(11) Publication number: **0 373 959 A2**

# (12) EUROPEAN PATENT APPLICATION

(21) Application number: 89313148.2

(22) Date of filing: 15.12.89

(51) Int. Cl.5: C07C 69/88, C07C 67/317

(30) Priority: 16.12.88 GB 8829399

(43) Date of publication of application: 20.06.90 Bulletin 90/25

(84) Designated Contracting States: AT BE CH DE ES FR GB GR IT LI LU NL SE

(71) Applicant: COURTAULDS PLC
18, Hanover Square
London W1A 2BB(GB)

(72) Inventor: Summers-Smith, Michael Alistair
37 Kenilworth Court
Coventry CV3 6HZ(GB)
Inventor: Coleman, Robert Jeffrey
10 Cecily Road, Cheylesmore
Coventry CV3 LA(GB)
Inventor: Stevens, Ian Edward
20 Douglas Road
Long Eaton Nottingham(GB)

(74) Representative: Hale, Stephen Geoffrey et al
J.Y. & G.W. Johnson Furnival House 14/18
High Holborn
London WC1V 6DE(GB)

## (54) Preparation of aromatic esters.

(57) A diester of 2,5-dihydroxybenzene-1,4-dicarboxylic acid is prepared by the oxidation of a diester of cyclohexane-1,4-dione-2,5-dicarboxylic acid. The oxidation is carried out by molecular oxygen in a non-aqueous polar solvent for the diester of cyclohexane-1,4-dione-2,5-dicarboxylic acid in the presence of a salt. The salt is preferably a transition metal salt which acts as a catalyst for the oxidation reaction and/or an alkali metal carboxylate.

EP 0 373 959 A2

## PREPARATION OF AROMATIC ESTERS

This invention relates to the preparation of diesters of 2,5-dihydroxybenzene-1,4-dicarboxylic acid, having the formula

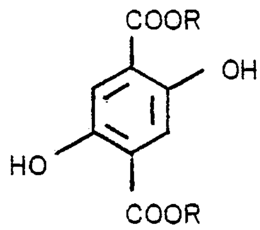

(I)

where R is an alkyl, aralkyl or aryl group. Such diesters are useful in the manufacture of polymers, especially polyesters. Polymers can be formed directly from (I), as described in GB-A-100194 and 1106568 and US-A-3244675, or (I) can be converted to a derivative in which the phenolic hydroxyl groups are etherified with long-chain alkyl groups before being polymerised, as described in M. Ballauff, Makromol. Chem. Rapid Commun., 1986, 7, at page 407.

The preparation of diethyl 2,5-dihydroxybenzene-1,4-dicarboxylate by oxidation of diethyl cyclohexane-1,4-dione-2,5-dicarboxylate using bromine in carbon disulphide is described by Herrmann in Annalen 211 at pages 327 and 355. Oxidation of the same starting material using bromine in chloroform is described by Ansell and Culling in J. Chem. Soc., 1961, at page 2908. The oxidation of the same starting material using thiourea is described by Taschner in Roczniki Chem., 1957, 25, at page 329.

The oxidation of diethyl cyclohexane-1,4-dione-2,5-dicarboxylate to 2,5-dihydroxybenzene-1,4-dicarboxylic acid using molecular oxygen in aqueous alkaline solution is described by Herrmann in Berichte. 1877, 10, at page 111. This oxidation reaction is also mentioned in DE-C-900495.

There is a need for improved preparation of diesters of formula (I). The diester is generally more useful than the diacid in subsequent reactions, especially if the phenol groups are to be etherified before polymerisation.

A process according to the invention for the preparation of a diester of 2,5-dihydroxybenzene-1,4-dicarboxylic acid, having the formula

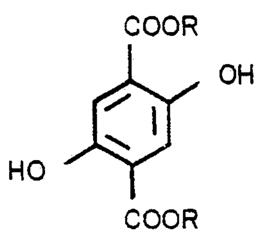

(I)

by the oxidation of a diester of cyclohexane-1,4-dione-2,5-dicarboxylic acid, having the formula

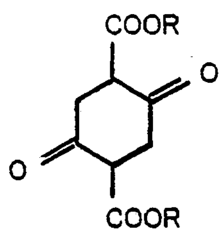

(II)

where R is an alkyl, aralkyl or aryl group, e.g. of up to 10 carbon atoms, is characterised in that the oxidation is carried out by molecular oxygen in a non-aqueous polar solvent for the diester of cyclohexane-1,4-dione-2,5-dicarboxylic acid in the presence of a salt.

The compound of formula (II) can be readily prepared from a succinic acid diester of the formula

ROOC $CH_2$ $CH_2$ COOR

by treatment with a sodium alkoxide in alcohol, as described in Organic Syntheses, Collective Volume 5 (published John Wiley & Sons, 1973) at page 288. For most purposes the group R in formulae (II) and (I) is preferably an alkyl group having 1 to 4 carbon atoms, for example an ethyl or methyl group. Other groups R such as hexyl, benzyl or phenyl can be used but are less preferred.

The molecular oxygen is preferably supplied in the form of air which is passed through the reaction solution. Oxygen-enriched air can be used. For best yields and purity of product it is preferred that carbon dioxide is removed from the air, for example by contacting the air with an alkali before it is passed through the reaction solution.

The preferred solvent for the reaction is a carboxylic acid, for example acetic acid or propionic acid. Alternative solvents are alcohols such as pentanol or butanol or aprotic polar solvents such as dimethylformamide or N-methylpyrrolidone. The temperature of reaction is preferably in the range 50 - 120 °C. The solvent should preferably have low volatility at the temperature of reaction. Use of a non-aqueous solvent avoids acid formation as described by Herrmann. The reaction is carried out in the substantial absence of water, but it is generally not necessary to remove water formed during the reaction.

The salt present in the reaction medium is preferably a transition metal salt which acts as a redox catalyst for the oxidation reaction. The transition metal catalyst is preferably a copper, iron, chromium or manganese salt. The transition metal is preferably in one of two adjacent oxidation states capable of stable existence, for example $Fe^{+++}$(ferric) or $Fe^{++}$(ferrous), $Cu^{++}$(cupric) or $Cu^{+}$(cuprous), $Mn^{+++}$ or $Mn^{++}$ or $Cr^{+++}$ or $Cr^{++}$. Iron and copper salts are particularly preferred, for example ferric chloride, cupric sulphate or cuprous chloride. The catalyst is preferably used at 0.2 to 10% by weight based on (II), most preferably 0.5 to 5% by weight.

Alternatively, a salt of an alkali metal or alkaline earth metal which is soluble in the reaction medium can be used. This is preferably a salt of a weak acid such as a carboxylic acid, for example, an alkali metal carboxylate such as sodium acetate used at 1-500%, preferably 20-150%, by weight, based on (II). A salt of a strong acid, for example sodium chloride or sodium sulphate, can be used but is less effective in promoting the oxidation than a carboxylate salt such as sodium acetate.

A salt of an anion which is a redox catalyst can be used, for example an iodide such as potassium iodide or sodium iodide. Alternatively, an alkali metal or alkaline metal salt, preferably a carboxylate salt such as sodium acetate, can be used in conjunction with an organic or inorganic redox catalyst such as hydroquinone or iodine. Most preferably the alkali metal salt (or possibly an alkaline earth metal salt) is present in the reaction mixture as well as a transition metal salt redox catalyst.

One advantage of carrying out the reaction in a carboxylic acid as solvent is that the product diester (I) is substantially insoluble in the carboxylic acid solvent at temperatures below 20-30 °C and crystallises out of the reaction mixture on cooling. It can therefore be collected by filtration. This leads to improved product purity as well as ease of recovery. Recovery of high purity product is particularly easy when a high conversion of (II) into (I) is achieved, for example when a copper salt catalyst and an alkali metal carboxylate are both present, since contamination of the crystallised product with starting material is avoided. Further product can be recovered by distillation or evaporating of solvents; alternatively, solvent containing some dissolved product and starting material can be reused.

The invention is illustrated by the following Examples.

## Example 1

Diethyl cyclohexane-1,4-dione-2,5-dicarboxylate (2.55 g), anhydrous sodium acetate (2.51 g), anhydrous cupric sulphate (30 mg) and glacial acetic acid (30 ml) were placed in a 100 ml reaction flask, and heated to 70-75 °C. Atmospheric air was drawn through the solution by suction, the inlet line being protected with a guard tube containing sodium hydroxide pellets to remove carbon dioxide, and the outlet line passing by way of a water-cooled condenser and a cold trap to minimise the loss of acetic acid by evaporation. Passage of air was continued for 5 hours, after which acetic acid was removed by distillation. The solid residue was slurried in water to dissolve metal salts, collected by filtration, washed with water and dried, to yield 2.35 g yellow crystals. $^1H$ NMR analysis showed that these consisted of over 95% diethyl 2,5-dihydroxybenzene-1,4-dicarboxylate, corresponding to a yield of 88%.

## Example 2

Example 1 was repeated, but with the use of cuprous chloride (20 mg) instead of cupric sulphate. 2.22 g crystals were collected; $^1H$ NMR showed these to contain at least 99% of the desired product, corresponding to a yield of 87%.

## Example 3

Example 1 was repeated, but with the use of ferric chloride (33 mg) instead of cupric sulphate. 2.23 g green crystals were collected; $^1H$ NMR analysis showed these to contain at least 99% of the desired product, corresponding to a yield of 88%.

## Example 4

Example 1 was repeated, but with the use of 50 mg of cupric sulphate as catalyst, and passage of air for only 2 hours. 2.35 g product were obtained. $^1H$ NMR analysis demonstrated that it consisted of 62% of the desired product and 38% unchanged starting diester.

## Example 5

Example 4 was repeated, but with the use of 104 mg of cupric sulphate as catalyst. 2.25 g product were obtained. $^1H$ NMR analysis showed that it contained 84% product and 16% unchanged diester, corresponding to a yield of 74%.

## Example 6

Example 1 was repeated, but without the use of the cupric sulphate catalyst. 2.00 g product were obtained. $^1H$ NMR analysis showed that it consisted of 25% of the desired product and 75% unchanged starting diester.

## Example 7

Example 1 was repeated, but with the use of only 0.82 g sodium acetate. 2.26 g crystals were collected; $^1H$ NMR analysis showed these to contain at least 99% of the desired product, corresponding to a yield of 89%.

## Example 8

Example 1 was repeated, but with the use of only 0.20 g sodium acetate. 2.24 g crystalline product were collected; $^1H$ NMR showed it to consist of 95% of the desired product and 5% unchanged starting diester, corresponding to a yield of 84%.

## Example 9

Example 1 was repeated, but without the use of sodium acetate. 2.11 g crystalline product were collected; $^1H$ NMR analysis showed it to consist of 21% of the desired product and 79% unchanged starting diester, corresponding to a yield of 17%.

## Example 10

Example 1 was repeated, but with the use of chromium (III) chloride hexahydrate (50 mg) instead of cupric sulphate. 2.15 g crystalline product were collected; $^1H$ NMR showed it to consist of 45% of the

desired product and 55% unchanged starting diester, corresponding to a yield of 38%.

**Example 11**

Example 1 was repeated, but with the use of manganese (II) chloride tetrahydrate (40 mg) instead of cupric sulphate. 2.11 g crystalline product were collected; $^1H$ NMR showed it to consist of 56% of the desired product and 44% unchanged starting diester, corresponding to a yield of 46%.

**Example 12**

Example 1 was repeated, but with the use of propionic acid (30 ml) instead of acetic acid as solvent. 2.42 g crystals were collected; $^1H$ NMR analysis showed these to contain at least 99% of the desired product, corresponding to a yield of 95%.

**Example 13**

Example 1 was repeated, but with the use of 1-butanol (30 ml) instead of acetic acid as solvent. 2.28 g crystal line product were collected; $^1H$ NMR analysis showed it to consist of 40% of the desired product and 60% unchanged starting diester, corresponding to a yield of 36%.

**Example 14**

Example 1 was repeated, but with the use of N,N-dimethylformamide (30 ml) instead of acetic acid as solvent. 1.89 g crystals were collected; $^1H$ NMR analysis showed these to contain at least 99% of the desired product, corresponding to a yield of 74%.

**Example 15**

Example 1 was repeated, but using 51.2 g of the diester, 51.8 g sodium acetate, 600 mg cupric sulphate, and 450 ml acetic acid. Passage of air was continued for a total of 14 hours, until NMR analysis showed that all starting diester had been consumed. Yellow crystals which separated during cooling were collected, washed with water, and dried; they weighed 42.6 g (84% of theory), and NMR analysis showed them to consist of at least 99% diethyl 2,5-hydroxybenzene-1,4-dicarboxylate.

If desired, the product can be purified further by recrystallisation from 2 volumes of ethyl acetate or from 3 volumes of acetic acid.

**Example 16**

Example 1 was repeated, but with the use of sodium chloride (1.85 g) instead of sodium acetate. 2.02 g product were collected; $^1H$ NMR analysis showed that this consisted of 37% product and 63% unchanged diester, corresponding to a yield of 29%.

**Example 17**

Example 1 was repeated, but with the use of anhydrous sodium sulphate (2.99 g) instead of sodium acetate. 2.02 g product were collected; $^1H$ NMR analysis showed that this consisted of 39% product and 61% unchanged diester, corresponding to a yield of 31%.

**Example 18**

Example 1 was repeated, but with the use of potassium iodide (33 mg) instead of cupric sulphate. 2.35 g product were collected; $^1H$ NMR analysis showed that this consisted of 74% product and 26% unchanged diester, corresponding to a yield of 68%.

## Example 19

Example 1 was repeated, but with the use of hydroquinone (22 mg) instead of cupric sulphate. 2.09 g product were collected; NMR showed that it consisted of 43% product and 57% unchanged diester, corresponding to a yield of 35%.

## Example 20

Example 1 was repeated, but using 102.4 g of the diester, 32.8 g sodium acetate, 1.23 g iron (III) chloride, and 500 ml acetic acid. Passage of air was continued for 19 hours, after which the solution was decanted from a small amount of insoluble material. The yellow crystalline product which separated on cooling was collected by filtration on a coarse sinter, washed with cold glacial acetic acid and water, and dried in vacuo at 70 °C. It weighed 79.1 g (78% of theory), and was shown by NMR analysis to consist of at least 99% of the desired product.

## Claims

1. A process for the preparation of a diester of 2,5-dihydroxybenzene-1,4-dicarboxylic acid, having the formula:

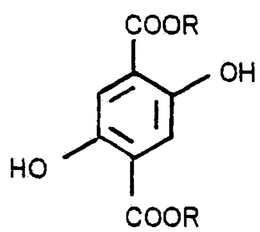

(I)

by the oxidation of a diester of cyclohexane-1,4-dione-2,5-dicarboxylic acid, having the formula:

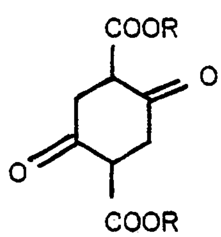

(II)

where R is an alkyl, aralkyl or aryl group, characterised in that the oxidation is carried out by molecular oxygen in a non-aqueous polar solvent for the diester of cyclohexane-1,4-dione-2,5-dicarboxylic acid in the presence of a salt.

2. A process according to claim 1, characterised in that the group R is an alkyl group having 1 to 4 carbon atoms.

3. A process according to claim 1 or claim 2, characterised in that the non-aqueous solvent is a carboxylic acid.

4. A process according to claim 3, characterised in that the carboxylic acid is acetic acid or propionic acid.

5. A process according to any of claims 1 to 4, characterised in that the temperature of reaction is 50-

120° C.

6. A process according to any of claims 1 to 5, characterised in that the said salt is a salt of a transition metal which is in one of two adjacent oxidation states capable of stable existence.

7. A process according to claim 6, characterised in that the salt is a copper or iron salt.

8. A process according to any of claims 1 to 5, characterised in that the said salt is an alkali metal carboxylate.

9. A process according to claim 8, characterised in that the alkali metal carboxylate is sodium acetate.

10. A process according to claim 8 or claim 9, characterised in that a redox catalyst is present in the reaction mixture.

11. A process according to claim 10, characterised in that the redox catalyst is a transition metal salt as defined in claim 6.